# EUROPEAN PATENT APPLICATION

(11) **EP 3 915 571 A1**
(43) Date of publication of application: **01.12.2021**
(21) Application number: 20177611.9
(22) Date of filing: 29.05.2020
(51) Int. Cl.: A61K 38/22, A61K 9/00, A61K 9/08, A61K 38/26, A61K 38/28, A61P 3/10, A61P 5/48

(54) **PHARMACEUTICAL COMPOSITION IN THE FORM OF AN INJECTABLE AQUEOUS SOLUTION INCLUDING AT LEAST A RAPID ACTING INSULIN ANALOG AND A GLUCAGON SUPPRESSOR WITH PRANDIAL ACTION**

(71) Applicant: Adocia, 69003 Lyon (FR)
(72) Inventor: CHAN, You-Ping, 69360 TERNAY (FR)
(74) Representative: Tripoz, Inès

(57) **Abstract**

The invention relates to a composition in the form of an injectable aqueous solution, the pH of which is from 3.6 to 4.4, including at least a rapid acting insulin analog and at least one glucagon suppressor with prandial action.

In an embodiment, the glucagon suppressor with prandial action is selected from the group consisting of an amylin analog or an amylin receptor agonist or a GLP-1 analog or a GLP-1 receptor agonist (GLP-1 RA).

In an embodiment, the glucagon suppressor with prandial action is an amylin analog or an amylin receptor agonist.

In an embodiment, the glucagon suppressor peptide with prandial action is pramlintide.

## Description

The invention relates to therapies by injection of a composition including at least a human insulin analog, with rapid prandial action, and a glucagon suppressor, in particular with prandial action, for treating diabetes and for allowing the improvement of the control of postprandial hyperglycemia.

To date, type 1 diabetes patients use two types of insulin, short-acting prandial insulins for controlling glycemia at mealtime, and long-acting basal insulins for controlling glycemia throughout the day and night. Several types of short-acting insulins exist, which are characterized by their onset of action. For example, human insulin, referred to as regular insulin, has a delayed action in comparison to the so-called rapid acting insulin analogs such as insulin lispro (human insulin B28K, B29P, Humalog®) or insulin aspart (human insulin B28D, Novolog®) or insulin glulisine (human insulin B3K,B29E, Apidra®). Thus, human insulin has to be administered on average 30 minutes before the meal, while the insulin analogs can be administered 15 minutes before the meal or at mealtime. In addition, insulin analogs are considered to lead to a better control of post-prandial glycemia than human insulin, which explains why a very large majority of patients in Europe and in the United States today use rapid acting insulin analogs whose action is shorter than that of human insulin. The rate of absorption of these insulins remains, however, to be improved, namely for use in artificial pancreas set-up where the insulin is administered by a pump controlled by an algorithm and connected to a continuous glucose measurement.

More recent approaches to improve the absorption rate over the existing rapid acting insulin analogs described above have consisted in the use of functional excipients and/or vasodilators. In yet another context, the formulation of human insulin and insulin analogs at acidic pH has been described. The PCT application WO2007/104786 describes compositions comprising rapid acting insulin analogs A21G, B28D desB30, and A21G, B28E desB30 with pramlintide at pH 3.5. The application also describes rapid insulin compositions, in particular B28D (insulin aspart) at neutral pH and in the presence of a surfactant, and in particular of a glycerophosphate derivative, more particularly dimyristoyl glycerophosphoglycerol (DMPG), leading to stabilities measured by ThT much greater than the stabilities of compositions of rapid acting insulin analogs A21G, B28D, desB30 and A21G, B28E, desB30 at acidic pH. This application does not show any PKPD results of insulin and pramlintide.

Patent application WO2019/020820 by the applicant describes the use of human insulin A21G in combination with pramlintide at pH 4 with or without zinc to obtain stable compositions having regular insulin absorption profile and slower pramlintide absorption profile in the pig model. This application does not disclose rapid acting insulin and glucagon suppressor formulations.

Patent application CN102199206 describes the combination of insulin lispro A21G and glargine containing zinc to obtain a mixed rapid and slow absorption of insulin. However, the rapid acting absorption of the insulin was slower compared to insulin lispro alone.

There is still a need for a stable rapid acting insulin and glucagon suppressor preparation namely for a better glucose control at mealtime and also for use in artificial pancreas applications. The formulation should also comply to stability requirements that are at least 2 years at 2-8°C, 2 weeks at 30°C and compatibility for use in insulin pumps.

However, the control of glycemia ensured by these exogenous prandial insulins after consumption of a meal is not optimal, even in the case of rapid acting insulin analogs. This is in part associated with the fact that these patients, in contrast to healthy persons, produce glucagon after consumption of a meal, which leads to the removal of some of the glucose stored in the liver. This glucose production mediated by glucagon worsens the post-prandial hyperglycemia problem of these patients and leads to excessive use of insulin.

This problem of regulation of post-prandial glycemia is rather similar for type 2 diabetes patients treated with insulin, in cases in which their disease has resulted in a very significant loss of their ability to produce insulin and amylin.

It has been demonstrated that glucagon suppressors, in particular peptides and/or hormones, are capable of inhibiting glucagon production after consumption of a meal, which leads to a significant improvement of the control of post-prandial glycemia.

Furthermore, it is particularly interesting that the rate of absorption of the insulin is closer to the one of an amylin analog or an amylin receptor agonist, such as pramlintide, to mimic better the co-secretion of insulin and amylin which is observed physiologically.

Surprisingly, the applicant has demonstrated that a composition containing rapid acting insulin analogs with A21 substitution, prepared at a pH range of 3.6 to 4.4 with a glucagon suppressor, in particular with an amylin agonist or an amylin receptor agonist, and more particularly with pramlintide, leads to a stable co-formulation with an effect of insulin and said glucagon suppressor close to physiological mechanism.

When the glucagon suppressor is an analog or a receptor agonist of amylin this can be measured, for example, by comparing the difference of tmax between the rapid acting insulin analog with A21 substitution and the tmax of the analog or a receptor agonist of amylin administered together in the same composition.. Stability studies also demonstrated both physical and chemical stabilities and satisfying US and EP requirements for liquid insulin products for subcutaneous injection by syringes or via pumps are obtained.

In order to obtain chemically and physically stable liquid formulations, there is a need to prepare formulations at an optimal pH. This pH range was found to be between 3.6 and 4.4, and more specifically at around pH 3.8. Under these conditions, rapid acting insulins for example insulin glulisine, insulin aspart or insulin aspart derivatives, as disclosed in WO2007/104786, are less soluble at pH 4.0 than at neutral pH and are hence not usable. On the other hand, insulin glargine is soluble at pH 4 but precipitate at neutral pH, which leads to its slow absorption after injection.

The applicant demonstrates that the combination with a glucagon suppressor with prandial action at a pH ranging from 3.6 to 4.4 in an aqueous solution enables to obtain an absorption profile of insulin and glucagon suppressor which is close to the physiological co-secretion of insulin and amylin.

The obtention of a pharmaceutical composition in the form of an injectable aqueous solution exhibiting such a property at these acidic pH and still maintained good physical and chemical stability properties in comparison to those described in the prior art is remarkable. As described above, the literature teaches away from the claimed composition.

According to an embodiment, the pharmaceutical composition in the form of an injectable aqueous solution is free of zinc or magnesium. By "free of zinc", is meant an amount of zinc on a weight/weight ratio which is less than 200 ppm and preferably less than 100 ppm.

By "free of magnesium", is meant an amount of magnesium on a weight/weight ratio which is less than 200 ppm and preferably less than 100 ppm.The invention relates to a pharmaceutical composition in the form of an injectable aqueous solution, the pH of which is from 3.6 to 4.4, including at least a rapid acting insulin analog with substitution of the residue on the position A21 and at least one glucagon suppressor with prandial action.

The invention relates to a pharmaceutical composition in the form of an injectable aqueous solution, the pH of which is from 3.6 to 4.4, including at least one glucagon suppressor with prandial action and at least a rapid acting insulin analog having the human insulin sequence with at least one substitution on the position A21 and another one. This means that the sequence of the rapid acting insulin analog comprises at least 2 differences with the sequence of the human insulin, one difference being at the position A21.

The invention also relates to a pharmaceutical composition in the form of an injectable aqueous solution, the pH of which is from 3.6 to 4.4and comprising at least one glucagon suppressor with prandial action and at least a rapid acting insulin analog having the human insulin sequence with at least the following substitutions:
- B28K,
- B29P and
- A21.

Substitution on the A21 position meaning that at this position the residue is not Asparagine (N).

According to an embodiment, the substitution at A21 is glycine, alanine, leucine, phenylalanine or serine.

According to another embodiment, the substitution on the position A21 is glycine. This substitution is also written "A21G".

By "rapid acting insulins", is meant insulins that are modified to increase hexamer dissociation.

In an embodiment, the rapid acting insulin is human insulin B28K, B29P with a substitution at position A21. The human insulin B28K, B29P is also called insulin lispro.

According to a preferred embodiment, the rapid acting insulin analog is insulin lispro A21G, also called human insulin A21G, B28K, B29P. This insulin lispro A21G comprises only these 3 substitutions as compared with human insulin.

According to another embodiment, the insulin analog is insulin lispro A21G, des B30 also called human insulin A21G, B28K, B29P, desB30. This insulin lispro A21G, desB30 comprises only these 4 substitutions as compared with human insulin.

According to another embodiment the rapid acting insulin analog with substitution of the residue on the position A21 is not desB30. In other words, the insulin comprises a residue at position B30. The residue at position B30 residue may be threonine (T).

According to an embodiment, the glucagon suppressor with prandial action is an amylin analog or an amylin receptor agonist, a GLP-1 analog or a GLP-1 receptor agonist, also referred to as GLP-1 RA.

The applicant has observed that the formulation according to the invention at a pH ranging from 3.6 to 4.4 has favorable pharmacokinetic properties and stability, in particular compatible with use in a pump for infusion. Such infusion may be subcutaneous infusion.

In an embodiment the pH is ranging from 3.7 to 4.3.

In another embodiment the pH is ranging from 3.8 to 4.2.

In another embodiment the pH is 3.8.

The applicant has observed that the pharmaceutical composition according to the invention at a pH from 3.6 to 4.4 has pharmacokinetic properties compatible with use at mealtimes and enables a better control of post-prandial glycemia.

The requirements that enable to obtain a pharmaceutical composition in the form of an injectable aqueous solution for diabetes treatment are in particular:
- an aqueous liquid formulation which shows a good physically and chemically stability, e.g. which is stable for at least one year or even 2 years at 5 °C, and at least two weeks at 30 °C (multiple uses),
- a compatibility with the antimicrobial preservatives.

Likewise, the pharmaceutical compositions of rapid acting insulin analog with A21 substitution with an amylin analog or an amylin receptor agonist, such as pramlintide, a GLP-1 analog or a GLP-1 receptor agonist, also referred to as GLP-1 RA, for example, exenatide or lixisenatide, at a pH from 3.6 to 4.4, have a physical and chemical stability enabling the development of a liquid formulation which is stable for at least one year or even 2 years at 5 °C and for at least 2 weeks at 30 °C.

In an embodiment, the invention relates to a pharmaceutical composition in the form of an injectable aqueous solution, the pH of which is from 3.6 to 4.4, including at least insulin lispro A21G and at least one glucagon suppressor with prandial action. In one embodiment this composition is free of zinc.

In another embodiment the pharmaceutical composition in the form of an injectable aqueous solution, has a pH ranging from 3.7 to 4.3.

In another embodiment the pharmaceutical composition in the form of an injectable aqueous solution, has a pH ranging from 3.8 to 4.2.

In another embodiment the pharmaceutical composition in the form of an injectable aqueous solution, has a pH of 3.8.

The pharmaceutical compositions in the form of an injectable aqueous solution according to the invention are clear solutions. "Clear solution" is understood to mean compositions which satisfy the criteria described in the American and European pharmacopoeias concerning the injectable solutions. In the US pharmacopoeia, the solutions are defined in part <1151> referring to the injection (<1>) (referring to <788> according to USP 35 and specified in <788> according to USP 35 and in <787>, <788> and <790> USP 38 (from August 1, 2014), according to USP 38). In the European pharmacopoeia, the injectable solutions have to meet the criteria given in sections 2.9.19 and 2.9.20.

In an embodiment, the invention relates to pharmaceutical composition in the form of an injectable aqueous solution, the pH of which is from 3.6 to 4.4, including at least insulin lispro A21G and pramlintide. In one embodiment this composition is free of zinc.

In an embodiment, the invention relates to a pharmaceutical composition in the form of an injectable aqueous solution, the pH of which is from 3.6 to 4.4, including at least insulin lispro A21G, a GLP-1 RA, such as exenatide or lixisenatide. In one embodiment this composition is free of zinc.

In an embodiment, the invention relates to a pharmaceutical composition in the form of an injectable aqueous solution, the pH of which is from 3.7 to 4.3, including at least insulin lispro A21G and pramlintide. In one embodiment this composition is free of zinc.

In an embodiment, the invention relates to a pharmaceutical composition in the form of an injectable aqueous solution, the pH of which is from 3.7 to 4.3, including at least insulin lispro A21G, a GLP-1 RA, such as exenatide or lixisenatide. In one embodiment this composition is free of zinc.

In an embodiment, the invention relates to pharmaceutical composition in the form of an injectable aqueous solution, the pH of which is from 3.8 to 4.2, including at least insulin lispro A21G and pramlintide. In one embodiment this composition is free of zinc.

In an embodiment, the invention relates to a pharmaceutical composition in the form of an injectable aqueous solution, the pH of which is from 3.8 to 4.2, including at least insulin lispro A21G, a GLP-1 RA, such as exenatide or lixisenatide. In one embodiment this composition is free of zinc.

In an embodiment, the invention relates to a pharmaceutical composition in the form of an injectable aqueous solution, the pH of which is 3.7, including at least insulin lispro A21G and pramlintide. In one embodiment this composition is free of zinc.

In an embodiment, the invention relates to a pharmaceutical composition in the form of an injectable aqueous solution, the pH of which is 3.7, including at least insulin lispro A21G, a GLP-1 RA, such as exenatide or lixisenatide. In one embodiment this composition is free of zinc.

In an embodiment, the invention relates to a pharmaceutical composition in the form of an injectable aqueous solution, the pH of which is 3.8, including at least insulin lispro A21G and pramlintide. In one embodiment this composition is free of zinc.

In an embodiment, the invention relates to a pharmaceutical composition in the form of an injectable aqueous solution, the pH of which is 3.8, including at least insulin lispro A21G, a GLP-1 RA, such as exenatide or lixisenatide. In one embodiment this composition is free of zinc.

In an embodiment, the concentration of the rapid acting insulin analog with an A21 substitution is from 600 to 3000 µM or from 100 to 500 U/mL.

In an embodiment, the concentration of the rapid acting insulin analog with an A21 substitution is from 600 to 1200 µM or from 100 to 200 U/mL.

In an embodiment, the concentration of the rapid acting insulin analog with A21 substitution is 600 µM or 100 U/mL.

In an embodiment, the concentration of the rapid acting insulin analog with A21 substitution is 1200 µM or 200 U/mL.

In an embodiment, the concentration of the rapid acting insulin analog with A21 substitution is from 2 to 20 mg/mL.

In an embodiment, the concentration of the rapid acting insulin analog with A21 substitution is from 3.5 to 10.5 mg/mL.

In an embodiment, the concentration of the rapid acting insulin analog with A21 substitution is 3.5 mg/mL.

In an embodiment, the invention relates to a pharmaceutical composition in the form of an injectable aqueous solution, the pH of which is from 3.6 to 4.4, including at least rapid acting insulin analog with A21 substitution and at least one glucagon suppressor with prandial action and is free of protamine salt.

In an embodiment, the invention relates to a pharmaceutical composition in the form of an injectable aqueous solution, the pH of which is from 3.6 to 4.4, including at least insulin lispro A21G and at least one glucagon suppressor with prandial action and is free of protamine salt.

In another embodiment, the invention relates to a pharmaceutical composition in the form of an injectable aqueous solution, the pH of which is from 3.6 to 4.4, including at least a rapid acting insulin analog with A21 substitution and at least one glucagon suppressor with prandial action and is free of protamine salt or other positively charged peptides comprising a percentage of positively charged amino acids which is greater than or equal to 40%.

In another embodiment, the invention relates to a pharmaceutical composition in the form of an injectable aqueous solution, the pH of which is from 3.6 to 4.4, including at least insulin lispro A21G and at least one glucagon suppressor with prandial action and is free of protamine salt or other positively charged peptides comprising a percentage of positively charged amino acids which is greater than or equal to 40%.

In an embodiment, the invention relates to a pharmaceutical composition in the form of an injectable aqueous solution, the pH of which is from 3.6 to 4.4, including at least insulin lispro A21G and at least one glucagon suppressor with prandial action and is free of insulin glargine.

In an embodiment the pharmaceutical composition according to the invention does not contain grafted insulin.

In an embodiment the pharmaceutical composition according to the invention does not contain acylated insulin.

In an embodiment the pharmaceutical composition according to the invention does not contain pegylated insulin.

In an embodiment, the invention relates to a pharmaceutical composition in the form of an injectable aqueous solution, the pH of which is between 3.6 to 4.4, including at least an insulin analog with the A21 substitution and amylin receptor agonist or insulin analog. According to an embodiment, said amylin receptor agonist or insulin analog is pramlintide.

In an embodiment, the invention relates to a pharmaceutical composition in the form of an injectable aqueous solution, the pH of which is between 3.7 to 4.3, including at least an insulin analog with the A21 substitution and amylin receptor agonist or insulin analog. According to an embodiment, said amylin receptor agonist or insulin analog is pramlintide.

In an embodiment, the invention relates to a pharmaceutical composition in the form of an injectable aqueous solution, the pH of which is between 3.8 to 4.2, including at least an insulin analog with the A21 substitution and an amylin receptor agonist or insulin analog. According to an embodiment, said amylin receptor agonist or insulin analog is pramlintide.

In an embodiment, the invention relates to a pharmaceutical composition in the form of an injectable aqueous solution, the pH of which is 3.8, including at least an insulin analog with the A21 substitution and an amylin receptor agonist or and insulin analog. According to an embodiment, said amylin receptor agonist or insulin analog is pramlintide.

In the present application, as mentioned, amylin refers to the compounds described in the patents US 5,124,314 and US 5,234,906. When the term "analog" is used, it refers to a peptide or a protein in which one or more constitutive amino acid residues of the primary sequence have been substituted by other amino acid residues and/or in which one or more constitutive amino acid residues have been eliminated and/or in which one or more constitutive amino acid residues have been added. The percentage of homology that is accepted for the present definition of an analog is 50%. In the case of amylin, an analog can be, for example, derived from the primary amino acid sequence of amylin by substituting one or more natural or non-natural or peptidomimetic amino acids.

In an embodiment, the invention relates to a pharmaceutical composition in the form of an injectable aqueous solution, the pH of which is between 3.6 to 4.4, including at least a rapid acting insulin analog with the A21 substitution and a GLP-1 receptor agonist or a GLP-1 analog. According to an embodiment, said GLP-1 receptor agonist is exenatide. According to another embodiment, said GLP-1 receptor agonist is lixisenatide.

In an embodiment, the invention relates to a pharmaceutical composition in the form of an injectable aqueous solution, the pH of which is between 3.7 to 4.3, including at least a rapid acting insulin analogs with A21 substitution, and free of zinc or magnesium ions and a GLP-1 receptor agonist or a GLP-1 analog. According to an embodiment, said GLP-1 receptor agonist is exenatide. According to another embodiment, said GLP-1 receptor agonist is lixisenatide.

In an embodiment, the invention relates to a pharmaceutical composition in the form of an injectable aqueous solution, the pH of which is between 3.8 to 4.2, including at least a rapid acting insulin analog with the A21 substitution and a GLP-1 receptor agonist or a GLP-1 analog. According to an embodiment, said GLP-1 receptor agonist is exenatide. According to another embodiment, said GLP-1 receptor agonist is lixisenatide.

In an embodiment, the invention relates to a pharmaceutical composition in the form of an injectable aqueous solution, the pH of which is 3.8, including at least a rapid acting insulin analog with the A21 substitution and a GLP-1 receptor agonist or a GLP-1 analog. According to an embodiment, said GLP-1 receptor agonist is exenatide. According to another embodiment, said GLP-1 receptor agonist is lixisenatide.

Exenatide and lixisenatide, which are described in the applications US2004/0023871 and WO0104156, respectively, are generally considered to be GLP-1 receptor agonists. In an embodiment, the glucagon suppressor with prandial action is pramlintide (Symlin®).

In an embodiment, the GLP-1, GLP-1 analogs, or GLP-1 RA are referred to as "short-acting" or "prandial." "Short-acting" or "prandial" is understood to mean GLP-1, GLP-1 analogs, or GLP-1 RA of which the apparent half-life of elimination after subcutaneous injection in humans is less than 8 hours, in particular less than 5 hours, preferably less than 4 hours or else less than 3 hours, such as, for example, exenatide or lixisenatide.

In an embodiment, the GLP-1, the GLP-1 analogs, or the GLP-1 RA are selected from the group consisting of exenatide (Byetta®), lixisenatide (Lyxumia®), the analogs or derivatives thereof and pharmaceutically acceptable salts thereof.

In an embodiment, the GLP-1, the GLP-1 analog, or GLP-1 RA is exenatide or Byetta®, analogs or derivatives thereof and pharmaceutically acceptable salts thereof.

In an embodiment, GLP-1, GLP-1 analog, or GLP-1 RA is lixisenatide or Lyxumia®, analogs or derivatives thereof and pharmaceutically acceptable salts thereof.

In an embodiment, the concentration of pramlintide is from 0.32 to 5 mg/mL.

In an embodiment, the concentration of pramlintide is from 0.5 to 1,5 mg/mL.

In an embodiment, the concentration of pramlintide is from 0.6 to 1.2 mg/mL.

In an embodiment, the concentration of pramlintide is from 0.6 to 1 mg/mL.

In an embodiment, the concentration of pramlintide is 1.0 mg/mL.

In an embodiment, the concentration of pramlintide is 0.6 mg/mL.

In an embodiment, the concentration of exenatide is from 10 to 1000 µg/mL.

In an embodiment, the concentration of exenatide is from 40 to 150 µg/mL.

In an embodiment, the concentration of exenatide is from 40 to 80 µg/mL.

In an embodiment, the concentration of exenatide is 50 µg/mL.

In an embodiment, the concentration of lixisenatide is from 20 to 1000 µg/mL.

In an embodiment, the concentration of lixisenatide is from 80 to 160 µg/mL.

In an embodiment, the concentration of lixisenatide is 100 µg/mL.

In an embodiment, the concentration of pramlintide is from 0.4 to 3 mg/mL for 100 U/ml of the rapid acting insulin with A21 substitution.

In an embodiment, the concentration of pramlintide is from 0.5 to 1,5 mg/mL for 100 U/ml of the rapid acting insulin with A21 substitution.

In an embodiment, the concentration of pramlintide is from 0.6 to 1.2 mg/mL for 100 U/ml of the rapid acting insulin with A21 substitution.

In an embodiment, the concentration of pramlintide is from 0.6 to 1 mg/mL for 100 U/ml of the rapid acting insulin with A21 substitution.

In an embodiment, the concentration of pramlintide is 1.0 mg/mL for 100 U/ml of the rapid acting insulin with A21 substitution.

In an embodiment, the concentration of pramlintide is 0.6 mg/mL for 100 U/ml of the rapid acting insulin with A21 substitution.

In an embodiment, the concentration of exenatide is from 10 to 1000 µg/mL for 100 U/ml of the rapid acting insulin with A21 substitution.

In an embodiment, the concentration of exenatide is from 40 to 150 µg/mL for 100 U/ml of the rapid acting insulin with A21 substitution.

In an embodiment, the concentration of exenatide is from 40 to 80 µg/mL for 100 U/ml of the rapid acting insulin with A21 substitution.

In an embodiment, the concentration of exenatide is 50 µg/mL for 100 U/ml of the rapid acting insulin with A21 substitution.

In an embodiment, the concentration of lixisenatide is from 20 to 1000 µg/mL for 100 U/ml of the rapid acting insulin with A21 substitution.

In an embodiment, the concentration of lixisenatide is from 80 to 160 µg/mL for 100 U/ml of the rapid acting insulin with A21 substitution.

In an embodiment, the concentration of lixisenatide is 100 µg/mL for 100 U/ml of the rapid acting insulin with A21 substitution.

In an embodiment, the pharmaceutical composition according to the invention does not contain a long acting insulin.

In an embodiment, the pharmaceutical composition according to the invention moreover includes buffers.

In an embodiment, the pharmaceutical composition according to the invention includes a buffer selected from the group consisting of a sodium acetate buffer and Tris.

In an embodiment, the pharmaceutical composition according to the invention moreover includes preservatives.

In an embodiment, the preservatives are selected from the group consisting of m-cresol and phenol, alone or in a mixture.

In an embodiment, the preservative is m-cresol.

In an embodiment, the concentration of m-cresol is ranging from 10 to 50 mM.

In an embodiment, the concentration of m-cresol is ranging from 10 to 40 mM.

In an embodiment, the preservative is phenol.

In an embodiment, the concentration of phenol is ranging from 10 to 60 mM.

In an embodiment, the concentration of phenol is ranging from 20 to 50 mM.

In an embodiment, the pharmaceutical composition according to the invention includes a surfactant.

In an embodiment, the surfactant is selected from the group consisting of Poloxamer 188, Tween® 20, also referred to as Polysorbate 20, and Tween® 80, also referred to as Polysorbate 80.

In an embodiment, the Tween® 20 concentration varies from 5 to 50 µg/mL.

In an embodiment, the Tween® 20 concentration varies from 5 to 25 µg/mL.

In an embodiment, the Tween® 20 concentration is 10 µg/mL.

In an embodiment, the Tween® 20 concentration is 10 µM.

The pharmaceutical composition according to the invention can moreover include additives such as tonicity agents.

In an embodiment, the tonicity agents are selected from the group consisting of glycerol, sodium chloride, mannitol and glycine.

In an embodiment, the pharmaceutical composition according to the invention moreover includes an antioxidant.

In an embodiment, the antioxidant is methionine.

In an embodiment, the pharmaceutical composition according to the invention contains 3.5 mg/mL to 10.5 mg/mL of insulin lispro A21G, 0.4 mg/mL to 3 mg/mL of pramlintide, 25 mM of m-cresol, 184 mM of glycerol at a pH of 3.8. This composition can moreover include polysorbate 20, in particular from 8 to 10 µM, and most particularly 8 µM.

In an embodiment, the pharmaceutical composition according to the invention contains 3.5 mg/mL of insulin lispro A21G, 0.6 mg/mL of pramlintide, 25 mM of m-cresol, 184 mM of glycerol, at a pH of 3.8. This composition can moreover include polysorbate 20, in particular 8 µM.

In an embodiment, the pharmaceutical composition according to the invention contains 3.5 mg/mL of insulin lispro A21G, 0.6 mg/mL of pramlintide, 25 mM of m-cresol, 184 mM of glycerol, 18 mM acetate buffer at a pH of 4.0. This composition can moreover include polysorbate 20, in particular 8 µM.

In an embodiment, the pharmaceutical composition according to the invention contains 7.0 mg/mL of insulin lispro A21G, from 0.8 to 2.0 mg/mL of pramlintide, 25 mM of m-cresol, 150 to 200 mM of glycerol, at a pH of 4,0. This composition can moreover include polysorbate 20, in particular from 8 to 10 µM, and most particularly 8 µM.

The pharmaceutical composition s according to the invention can moreover include excipients in compliance with the Pharmacopoeias, in particular the EP and/or US Pharmacopoeias, and compatible with the insulins used at the usual concentrations.

According to an embodiment, the pharmaceutical composition can be reconstituted from a solid or lyophilized form.

The methods of administration considered are the intravenous, subcutaneous, intradermal or intramuscular route.

According to a particular embodiment, the method of administration is the subcutaneous route.

In an embodiment, the pharmaceutical composition is administered in one or several boluses at mealtime.

In an embodiment, the pharmaceutical composition is administered in 2 boluses at mealtime.

In an embodiment, the pharmaceutical composition is administered in 3 boluses at mealtime.

By "at mealtime" is meant a time ranging from 20 minutes before to 30 minutes after the beginning of the meal. In particular, from 10 minutes before to 30 minutes after the beginning of the meal. More particularly 5 minutes before to 30 minutes after the beginning of the meal. Even more particularly 5 minutes before to 20 minutes after the beginning of the meal.

In an embodiment, the pharmaceutical composition, intended to be used in a diabetes treatment method, is administered to improve the control of postprandial glycemia and to decrease the adverse effects of pramlintide.

In an embodiment, the pharmaceutical composition, intended to be used in a diabetes treatment method, enables to decrease the food consumption induced by insulin.

The transdermal, oral, nasal, vaginal, ocular, buccal, pulmonary administration routes are also considered.

The invention also relates to an implantable or transportable pump including a pharmaceutical composition according to the invention.

The invention also relates to the use of a pharmaceutical composition according to the invention which is intended to be placed in an implantable or transportable pump.

The invention also relates to the use of a pharmaceutical composition according to the invention which is intended to be placed in a pump working with a closed loop system.

The invention also relates to the use of a pharmaceutical composition according to the invention which is intended to be placed in a patch pump.

The invention also relates to a cartridge comprising the pharmaceutical composition.

The invention also relates to a vial comprising the pharmaceutical composition.

The preparation of a pharmaceutical composition according to the invention has the advantage that it can be implemented by simple solubilization in water of an insulin analog and one glucagon suppressor with prandial action.

The preparation of a pharmaceutical composition according to the invention has the advantage that it can be implemented by simple solubilization of an amylin analog or of an amylin receptor agonist and insulin analog.

If necessary, the composition of the pharmaceutical composition is adjusted in terms of excipients such as glycerol, m-cresol and polysorbate 20 (Tween® 20). This addition can be carried out by addition of concentrated solutions of said excipients.

The rapid acting insulin analogs with A21 substitution can be obtained by methods of recombinant DNA technology using bacteria such as *Escherichia coli* and yeasts such as *Saccharomyces cerevisiae* (see, for example, G. Walsh Appl. Microbiol. Biotechnol. 2005, 67, 151-159) or by chemical synthesis. In general, a proinsulin is produced, which is then digested by enzymes such as trypsin and carboxypeptidase B to obtain the desired sequence.

For the production of rapid acting insulin analogs with A21 substitution, the proinsulin is coded with the chosen A21 aminoacid and the other substitutions, and, after digestion by trypsin and carboxypeptidase B, the desired insulin is obtained. An operating procedure is described by Kohn et al., in Peptides 2007, 28, 935-948.

The pharmaceutical composition in the form of an injectable aqueous solution according to the invention has a physical and chemical stability enabling the development of a liquid formulation which is stable for at least one year or even 2 years at 5 °C and for at least 2 weeks 30 °C. The stability being defined according to the EP and/or US Pharmacopeia.

With regard to the stability, a conventional accelerated method for measuring the physical stabilities of proteins or peptides consists in measuring the formation of fibrils with the aid of thioflavin T, also referred to as ThT. This method enables to measure, under temperature and stirring conditions enabling an acceleration of the phenomenon, the lag time before the formation of fibrils by measuring the increase in fluorescence. The compositions according to the invention have a lag time before the formation of fibrils that is clearly greater than those described in the literature in the same conditions. The compositions according to the invention exhibit a physical and chemical stability which is much greater than those described in the prior art by using commercial prandial insulins.

In an embodiment, pharmaceutical composition in the form of an injectable aqueous solution according to the invention exhibits a lag time measured by ThT equal to at least 6 hours.

In an embodiment, pharmaceutical composition according to the invention exhibits a lag time measured by ThT equal to at least 8 hours.

In an embodiment, the pharmaceutical composition according to the invention exhibits a lag time measured by ThT equal to at least 10 hours.

In an embodiment, the pharmaceutical composition according to the invention exhibits a lag time measured by ThT equal to at least 15 hours.

### Example 1:

### Preparation of a pharmaceutical composition comprising insulin lispro A21G 100 U/mL (3.5 mg/mL) and of pramlintide 0.6 mg/mL containing m-cresol (25 mM), glycerol (184 mM) and acetic acid/sodium acetate buffer (18 mM) at acidic pH of 3.8.

A concentrated solution of excipients (m-cresol, glycerol) is added to a concentrated solution of insulin lispro A21G in an acetic acid/sodium acetate buffer at pH 3.8. A concentrated solution of pramlintide (10 mg/mL at pH 3.8) is added to this concentrated solution of insulin lispro A21G and of excipients so as to obtain the intended final pharmaceutical composition. The final pH 3.8, is obtained by addition of an aqueous solution of NaOH. The solution obtained is clear and homogeneous; it is subjected to a 0.22 µm filtration and stored in glass cartridges (1 mL of solution per 3 mL cartridge).

### Example 2:

### Preparation of a pharmaceutical composition comprising insulin lispro A21G 100 U/mL (3.5 mg/mL) and of pramlintide 0.6 mg/mL containing m-cresol (25 mM), glycerol (184 mM), acetic acid/sodium acetate buffer (18 mM) and Tween 20 (10 µg/mL) at acidic pH of 3.8.

A concentrated solution of excipients (m-cresol, glycerol) is added to a concentrated solution of insulin lispro A21G in an acetic acid/sodium acetate buffer at pH 3.8. A concentrated solution of pramlintide. A concentrated solution of pramlintide (10 mg/mL at pH 3.8) and a concentrated solution of Tween 20 are added to this concentrated solution of insulin lispro A21G and of excipients so as to obtain the intended final pharmaceutical composition. The final pH 3.8, is obtained by addition of an aqueous solution of NaOH. The solution obtained is clear and homogeneous; it is subjected to a 0.22 µm filtration and stored in glass vials (1.2 mL of solution per 2 ml vial).

## Claims

1. A pharmaceutical composition in the form of an injectable aqueous solution, the pH of which is from 3.6 to 4.4 and comprising at least one glucagon suppressor with prandial action and at least a rapid acting insulin analog having the human insulin sequence with at least the following substitutions:.
- B28K,
- B29P,
- A21.

2. The pharmaceutical composition according to claim 1, **characterized in that** it is free of zinc.

3. The pharmaceutical composition according to claim 1 or 2, **characterized in that** the position A21 of the rapid acting insulin analog is glycine, alanine, leucine, phenylalanine or serine.

4. The pharmaceutical composition according to claim 1 or 2, **characterized in that** the rapid acting insulin analog is insulin lispro A21G, also called human insulin A21G,B28K,B29P.

5. The pharmaceutical composition according to claim 1 or 2, **characterized in that** the glucagon suppressor with prandial action is selected from the group consisting of an amylin analog or an amylin receptor agonist or a GLP-1 analog or a GLP-1 receptor agonist (GLP-1 RA).

6. The pharmaceutical composition according to any one of the preceding claims, **characterized in that** the glucagon suppressor with prandial action is an amylin analog or an amylin receptor agonist.

7. The pharmaceutical composition according to any one of the preceding claims, **characterized in that** the glucagon suppressor peptide with prandial action is pramlintide.

8. The pharmaceutical composition according to any one of the preceding claims, **characterized in that** the glucagon suppressor peptide with prandial action is exenatide.

9. The pharmaceutical composition according to any one of the preceding claims, **characterized in that** the glucagon suppressor peptide with prandial action is lixisenatide.

10. The pharmaceutical composition according to claim 4 to 9**characterized in that** the concentration of insulin lispro A21G is from 2 to 20 mg/mL.

11. The pharmaceutical composition according to claim 4 to 9, **characterized in that** the concentration of insulin lispro A21G is 3.5 mg/mL.

12. The pharmaceutical composition according to any one of the preceding claims, **characterized in that** the concentration of glucagon suppressor peptide with prandial action is from 0.01 to 10 mg/mL.

13. The pharmaceutical composition according to any one of the preceding claims, **characterized in that** the pH of the solution is from 3.7 to 4.3.

14. The pharmaceutical composition according to any one of the preceding claims, intended to be used in a diabetes treatment method, **characterized in that** it is administered as one or several boluses at mealtime.

15. The pharmaceutical composition according to any one of the preceding claims, intended to be used in a diabetes treatment method, **characterized in that** it is administered using a pump.

16. The pharmaceutical composition according to any one of claims 1 to 15, intended to be used in a diabetes treatment method, **characterized in that** it is administered to improve the control of postprandial glycemia.

17. The pharmaceutical composition according to any one of claims 1 to 15, intended to be used in a diabetes treatment method, **characterized in that** it is administered to improve the control of postprandial glycemia and to decrease the adverse effects of pramlintide.

18. The pharmaceutical composition according to any one of claims 1 to 15, intended to be used in a diabetes treatment method, **characterized in that** it enables to decrease the food consumption induced by insulin.
